# EUROPEAN PATENT SPECIFICATION

(11) **EP 2 410 845 B1**
(45) Date of publication and mention of the grant of the patent: **18.02.2015**
(21) Application number: 10722387.7
(22) Date of filing: 25.03.2010
(51) Int. Cl.: A01N 57/16, A01N 53/00, A01P 7/00

(54) **Compositions comprising azamethiphos and either cypermethrin or deltamethrin to combat ectoparasites in fish**
Zusammensetzungen enthaltend azamethiphos und entweder Cypermethrin oder Deltamethrin zur Bekämpfung von Ektoparasiten auf Fisch
Compositions comprenant azaméthiphos et soit cyperméthrine soit deltaméthrine pour la lutte contre les ectoparasites des poissons

(30) Priority: 25.03.2009 GB 0905165
(43) Date of publication of application: 01.02.2012
(73) Proprietor: Nettforsk AS, 4801 Arendal (NO)
(72) Inventor: JOHANNESSEN, Baard, N-4801 Arendal (NO)
(74) Representative: Hoffmann, Benjamin
(86) International application number: PCT/GB2010/000569
(87) International publication number: WO 2010/109198

(56) References cited:
- WO-A2-2009/010755
- FR-A1- 2 793 112
- SCHERING-PLOUGH ANIMAL HEALTH: "Sea Lice, Technical Monograph, SLICE" INTERNET CITATION 31 December 2002 (2002-12-31), pages 1-20, XP002558899 Retrieved from the Internet: URL:http://www.spaquaculture.com/assets/3- 28sealcfinal.pdf [retrieved on 2009-12-03]
- GRANT ANDREW N: "Medicines for sea lice" PEST MANAGEMENT SCIENCE, vol. 58, 7 May 2002 (2002-05-07), pages 521-527, XP002592569 ISSN: 1526-498X DOI: 10.1002/ps.481
- AHMAD M; SALEEM M A; SAYYED A H: "Efficacy of insecticide mixtures against pyrethroid- and organophosphate-resistant populations of Spodoptera litura (Lepidoptera: Noctuidae)" PEST MANAGEMENT SCIENCE, vol. 65, 2 December 2008 (2008-12-02), pages 266-274, XP007915228 DOI: 10.1002/ps.1681
- AHMAD M: "Observed potentiation between pyrethroid and organophosphorus insecticides for the management of Spodoptera litura (Lepidoptera: Noctuidae)" CROP PROTECTION, vol. 28, 13 December 2008 (2008-12-13), pages 264-268, XP025927811 ISSN: 0261-2194 DOI: 10.1016/j.cropro.2008.11.001

## Description

The present invention relates to topical pesticidal compositions for use in a method of pesticidal treatment of fish to combat infestation by exoskeletal ectoparasite species, in particular lice, insects, arachnids, etc.

In agriculture and aquaculture, the crop is frequently plagued by exoskeletal pests. For example, in aquaculture sea lice, in plant crops aphids and various insects, and in land animals fleas and lice. Likewise, humans may be infested by head lice and other exoskeletal pests.

Ectoparasites of the orders *Phthiraptera* (lice), *Acarina* (mites) and *Siphonaptera* (fleas), more especially *Pediculus humanus capitis* (human head lice), body louse (*Pediculus humanus humanus),* the pubic louse *(Pthirus pubis)* and the scabies mite *(Sarcoptes scabiei)* are of particular concern to humans. Ectoparasites of the order copepod crustaceans (sea lice), like *Lepeoptheirus spp* and *Caligus spp* (especially *Lepeoptheirus salmonis* and *Caligus elongatus*) are of particular concern for farmed fish. The desert locust and the beetle *Meligethes aeneus* are especially important pests for crop plants.

Typically, one seeks to eliminate such exoskeletal pests using organophosphates or pyrethroids. However, the pest species has an annoying tendency to become resistant to the organophosphate or pyrethroid being used and it becomes necessary to cycle between different biocides. Moreover such biocides, the organophosphates in particular, have been found to have detrimental side effects on higher life forms, in particular birds and humans, for example the appliers of the biocide or the consumers of the treated product or the treated pests. Since pyrethroids are less effective in warm climates, there is a tendency amongst farmers in warm regions to choose organophosphate biocides and as a result not only are their plant products so contaminated as to be unexportable to profitable markets but the pesticidal effect is diminished due to the side effects on the natural predators of the pest species, e.g. birds, reptiles, etc.

There have been proposals to treat simultaneously with a combination of a pyrethroid and an organophosphate or to treat first with one and then with the other so as to enhance the pesticidal efficiency. In general however such proposals involve the use of standard or only slightly below standard doses of both of the two pesticides and as a result the problems of organophosphates contamination or poisoning are barely reduced.

A method of treating farmed fish to combat infestation by multicellular ectoparasites with exoskeletons is disclosed in WO 2009/010755.

We have now found however that a combination, i.e. simultaneous, treatment with a pyrethroid and an organophosphate is surprisingly effective and involves little or no organophosphate contamination if the pyrethroid is administered at 10 to 120%, particularly 15 to 100%, especially 20 to 75%, of the normal dose and the organophosphate is administered at 0.05 to 3.5%, especially 0.1 to 3.0%, particularly 0.5 to 2.5% of the normal dose.

Thus viewed from one aspect the invention provides a topical pesticide composition for use in method of pesticidal treatment of fish, which method comprises applying to said fish by administration into water in which said fish are contained, the topical pesticidal composition such that the concentration of azamethiphos is 0.5 to 20 ppb by weight, wherein said composition comprises either cypermethrin and azamethiphos in a weight ratio of 1:10 to 5:1, or deltamethrin and azamethiphos in a weight ratio of 1:10 to 5:1.

Particularly preferably, animals treated with the compositions according to the invention are also treated with a CYP enzyme inhibitor as a synergist for the pyrethroid/pyrethrin, e.g. piperonyl butoxide. Especially preferably said animals are treated with a mixed function oxidase (MFO) inhibitor. This may be present in the compositions according to the invention or alternatively may be administered with the animal's feed, e.g. 12 hours to 60 hours, especially 24 to 48 hours, before exposure to the compositions of the invention.

Insofar as aquatic animals are concerned, the method of the invention is particularly applicable to farmed, vertebrate fish, e.g. salmon (and other salmonids), cod, etc. Farmed salmon in particular are especially prone to infestation by sea lice. For the treatment of fish, the pyrethroid concentration is preferably 0.3 to 30ppb (by wt), especially 0.5 to 20ppb, particularly 1 to 15ppb. The organophosphate concentration is 0.5 to 20ppb, preferably 1 to 15ppb, particularly 2 to 8ppb. The preferred concentration for deltamethrin is 2 ppb, while that for cypermethrin is 5-15 ppb.

The organophosphate biocide of the compositions of the invention is the conventional organophosphate biocide azamethiphos.

The pyrethroid biocide of the compositions of the invention may be either of the conventional pyrethroid biocides, cypermethrin (including alpha- and zeta-cypermethrin), or deltamethrin (also known as decamethrin).

Particularly preferred organophosphate and pyrethroid combinations include: azametiphos and deltamethrin ; azametiphos and cypermethrin.

The compositions of the invention will typically also include a solvent, for example water, an alcohol (e.g. a C₁₋₆ alcohol), or a water/alcohol mixture. If desired, a surfactant, a polyol or an oil may also be included to facilitate wetting or adhesion.

The concentration of the biocides in the compositions of the invention will depend on the manner of application - the more direct the mode of application, the higher the concentration can be as a general rule. Application is by administration into water containing the host species.

For topical treatment of lice the pyrethroid is preferably present in the composition at a concentration of 0.2 to 3% wt, especially 0.5 to 2% wt, particularly about 1% wt. The remaining components of the composition may be conventional components for topical compositions and may be present in conventional amounts, e.g. water, alcohol, gelling agents, surfactants, fragrances, etc.

The composition of the invention may if necessary be reapplied, e.g. after 7 to 10 days, but for a single case of infestation a single application of the composition will generally be sufficient.

Contact with the compositions of the invention is desirably maintained for 15 to 180 minutes at least, preferably 30 to 90 minutes, especially about 60 minutes.

The compositions according to the invention may advantageously contain a further biocide, e.g. selected from the chloronicotinyl (e.g. imidacloprid), phenylpyrazole (e.g. fipronil), oxadiazine (e.g. indoxacarb), pyrazole (e.g. chlorfenapyr), or organochlorine (e.g. lindane) classes. The phenylpyrazoles and organochlorines however are not preferred.

The compositions according to the invention may take any convenient topical application form, e.g. solution, dispersion, powder, etc. Since they will generally be diluted on or before application, their concentrations and formulations are not critical. Commercially available compositions may be admixed for use according to the methods and uses of the invention.

The compositions comprising a pyrethroid and an organophosphate described herein for use in therapy or medicine and for use in the methods herein described also form part of the invention. Viewed from a further aspect, the present invention therefore provides the use of a pyrethroid and an organophosphate in the manufacture of a medicament, preferably a topical medicament, for application to fish to combat infestation by exoskeletal ectoparasites wherein said composition comprises either cypermethrin and azamethiphos in a weight ratio of 1:10 to 5:1, or deltamethrin and azamethiphos in a weight ratio of 1:10 to 5:1, and wherein the concentration of azamethiphos is 0.5 to 20 ppb by weight.

The compositions as herein described for use in the methods of combating pests or pesticidal treatment of fish as herein described form a further embodiment of the invention.

The invention will now be described with reference to the following Examples.

### Example 1

### Composition for treatment of salmon

A combination is made of 5 parts wt. azametiphos and 5 or 15 parts wt. of cypermethrin (or 2 or 3 parts wt. deltamethrin) and 1 billion parts wt. aqueous carrier solution. The lower pyrethroid dose may be used for non-pyrethroid-resistant sea-lice. Application may be by immersion of the fish in the composition, e.g. for around 80 minutes, for example 30 to 70 minutes.

### Reference Example 2

### Composition for treatment of citrus crops

The compositions of Example 1 may be used, applied by spraying, e.g. to citrus trees.

### Reference Example 3

### Composition for the treatment of hair-bearing land animal

Azametiphos (in concentrated solution in isopropanol) is added at 0.5% wt and deltamethrin is added at 1% wt to a commercially available shampoo, e.g. Head & Shoulders from Proctor & Gamble, or cream rinse/conditioner. After application, rinsing should be delayed for 30 to 60 minutes. Treatment is desirably effected twice in one day.

## Claims

1. A topical pesticide composition for use in a method of pesticidal treatment of fish to combat infestation by ectoparasites, which method comprises applying said composition to said fish by administration into water in which said fish are contained such that the concentration of azamethiphos is 0.5 to 20 ppb by weight,
wherein said composition comprises
either
cypermethrin and azamethiphos in a weight ratio of 1:10 to 5:1,
or
deltamethrin and azamethiphos in a weight ratio of 1:10 to 5:1.

2. The composition as claimed in claim 1 for use in the pesticidal treatment of fish to combat infestation by *Lepeoptheirus salmonis* or *Caligus elongatus.*

3. The composition as claimed in claim 1 or claim 2 wherein said weight ratio is 1:3 to 3:1.

## Patentansprüche

1. Topische Pestizidzusammensetzung zur Verwendung bei einem Verfahren zur Behandlung von Fischen mit Pestiziden, um einen Befall durch Ektoparasiten zu bekämpfen, wobei das Verfahren eine solche Anwendung der Zusammensetzung an den Fischen durch die Verabreichung in das Wasser, in dem die Fische enthalten sind, umfasst, daß die Konzentration von Azamethiphos 0,5 bis 20 ppb, bezogen auf das Gewicht, beträgt,
wobei die Zusammensetzung entweder
Cypermethrin und Azamethiphos in einem Gewichtsverhältnis von 1:10 bis 5:1 umfasst,
oder
Deltamethrin und Azamethiphos in einem Gewichtsverhältnis von 1:10 bis 5:1 umfasst.

2. Zusammensetzung nach Anspruch 1 zur Verwendung bei der Behandlung von Fischen mit Pestiziden, um den Befall durch *Lepeoptheirus salmonis* oder *Caligus elongatus* zu bekämpfen.

3. Zusammensetzung nach Anspruch 1 oder Anspruch 2, wobei das Gewichtsverhältnis 1:3 bis 3:1 beträgt.

## Revendications

1. Composition de pesticides topiques, pour une utilisation dans un procédé de traitement par pesticide des poissons pour lutter contre une infestation par des ectoparasites, ledit procédé comprenant l'application de ladite composition sur lesdits poissons par administration dans l'eau dans laquelle lesdits poissons sont contenus, telle que la concentration en azaméthiphos est de 0,5 à 20 ppb en poids,
dans laquelle ladite composition comprend soit
de la cyperméthrine et de l'azaméthiphos à un rapport pondéral de 1/10 à 5/1,
soit
de la deltaméthrine et de l'azaméthiphos à un rapport pondéral de 1/10 à 5/1.

2. Composition selon la revendication 1, pour une utilisation dans le traitement par pesticide des poissons pour lutter contre une infestation par *Lepeoptheirus salmonis* ou *Caligus elongatus.*

3. Composition selon la revendication 1 ou la revendication 2, dans laquelle ledit rapport pondéral est de 1/3 à 3/1.
